# EUROPÄISCHE PATENTSCHRIFT

(11) **EP 1 590 491 B1**
(45) Veröffentlichungstag und Bekanntmachung des Hinweises auf die Patenterteilung: **01.06.2011**
(21) Anmeldenummer: 04703789.0
(22) Anmeldetag: 21.01.2004
(51) Int. Cl.: C14C 3/16, C14C 3/18, C07D 309/30

(54) **ADDUKTE AUF BASIS CYCLISCHER VERBINDUNGEN UND IHRE VERWENDUNG ALS GERBSTOFFE UND KONSERVIERUNGSMITTEL**
ADDUCTS BASED ON CYCLIC COMPOUNDS AND THE USE THEREOF AS TANNING AGENTS AND CURING AGENTS
PRODUITS D'ADDITION A BASE DE COMPOSES CYCLIQUES ET LEUR UTILISATION COMME AGENTS DE TANNAGE ET AGENTS DE CONSERVATION

(30) Priorität: 28.01.2003 DE 10303311
(43) Veröffentlichungstag der Anmeldung: 02.11.2005
(73) Patentinhaber: BASF SE, 67056 Ludwigshafen (DE)
(72) Erfinder: HÜFFER, Stephan, 67063 Ludwigshafen (DE); REESE, Oliver, 68219 Mannheim (DE); GRAMLICH, Walter, 68259 Mannheim (DE); SCHROEDER, Stefan, 67271 Neuleiningen (DE); SCHERR, Günter, 67065 Ludwigshafen (DE); BACH, Volker, 67434 Neustadt (DE)
(86) Internationale Anmeldenummer: PCT/EP2004/000454
(87) Internationale Veröffentlichungsnummer: WO 2004/067781

(56) Entgegenhaltungen:
- GB-A- 1 462 309
- L. LEPAGE, Y. LEPAGE: "Synthèse de dialdéhydes-1,5 diéniques par réaction en milieu acide, d'aldéhydes aromatiques avec le méthoxy-2 dihydro-3,4 2H-pyranne" BULL. SOC. CHIM. FRANCE, 1988, Seiten 591-594, XP002285012

## Beschreibung

Die vorliegende Erfindung betrifft Addukte, erhältlich durch Umsetzung von Carbonylverbindungen der allgemeinen Formel 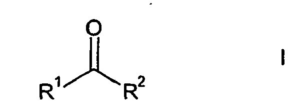 in denen die Variablen wie folgt definiert sind:
R¹, R² ausgewählt aus Wasserstoff, C₁-C₁₂-Alkyl, C₃-C₁₂-Cycloalkyl, substituiert oder unsubstituiert, C₇-C₁₃-Aralkyl, wobei R¹ und R² miteinander unter Bildung eines Rings verbunden sein können,
   mit cyclischen Verbindungen der allgemeinen Formel II, 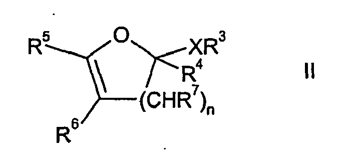
in denen die Variablen wie folgt definiert sind:
X ausgewählt aus Sauerstoff, Schwefel und N-R⁸,
R³, R⁸ gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, Formyl, CO-C₁-C₁₂-Alkyl, CO-C₃-C₁₂-Cycloalkyl, substituiert oder unsubstituiert, CO-C₇-C₁₃-Aralkyl, CO-C₆-C₁₄-Aryl, wobei für den Fall, dass X = N-R⁸ bedeutet, R³ und R⁸ unter Bildung eines Rings miteinander verbunden sein können;
R⁴, R⁵, R⁶ gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, wobei jeweils zwei benachbarte Reste unter Bildung eines Rings miteinander verbunden sein können;
n eine ganze Zahl im Bereich von 1 bis 4;
R⁷ gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, wobei R⁷ mit R⁶ oder auch jeweils zwei benachbarte Reste R⁷ miteinander unter Bildung eines Rings verbunden sein können.

Seit über 100 Jahren ist die Chromgerbung in der Lederherstellung eine wichtige chemische Behandlung, siehe beispielsweise Ullmann's Encyclopedia of Industrial Chemistry, Band A15, Seite 259 bis 282 und insbesondere Seite 268 ff., 5. Auflage, (1990), Verlag Chemie Weinheim. Aus ökologischen Gründen wird jedoch nach Alternativen zur Chromgerbung gesucht. Bei der herkömmlichen Chromgerbung werden Chromsalze in einer Menge von 1,5 bis 8 Gew.-%, bezogen auf das Blößengewicht des Leders, oder sogar mehr angeboten. Hiervon wird meist ein erheblicher Teil nicht gebunden und gelangt ins Abwasser. Zwar gelingt es, das Abwasser durch chemische Behandlung mit beispielsweise Kalk und Eisensalzen von beträchtlichen Anteilen Chrom zu befreien, es fallen jedoch Chrom-haltige Schlämme an, die auf Sonderdeponien entsorgt oder aufwändig aufgearbeitet werden müssen.

Außerdem fallen beispielsweise beim Spalten der Häute und beim Egalisieren der Leder Chrom-haltige Lederabfälle an, die etwa 8 bis 15 Gew.-%, bezogen auf das Hautgewicht, ausmachen können und ebenfalls aufwändig entsorgt werden müssen.

Es hat nicht an Versuchen gefehlt, durch beispielsweise Recyclieren der Chromgerbflotten oder Chromrecyclingverfahren die Chrombelastung der Abwässer zu verringern. Diese Verfahren waren aber im Ganzen unbefriedigend und insbesondere nicht in der Lage, das Problem der Chrom-haltigen Lederabfälle zu lösen.

Bekannt sind des weiteren Verfahren, in denen das Chrom ganz oder teilweise durch organische Gerbmittel ersetzt wurde. Genannt seien der Einsatz der sogenannten Syntane, das sind sulfonierte Kondensationsprodukte von Formaldehyd und Phenol oder sulfonierte Naphthalin-Formaldehyd-Kondensationsprodukte. Genannt sei weiterhin der Einsatz sogenannter vegetabiler Gerbstoffe. Beide Klassen von Gerbmitteln sorgen jedoch für einen erhöhten CSB-Wert der Abwässer und sind aus Umweltgründen ebenfalls nicht unbedenklich. Außerdem hat sich erwiesen, dass die Lichtechtheit der Leder bei der Verwendung von sulfonierten Phenol/Formaldehyd-Kondensationsprodukten oft zu wünschen übrig lässt (Ullmann's Encyclopedia of Industrial Chemistry, Band A15, Seite 259 bis 282 und insbesondere Seite 270 ff.,5. Auflage, (1990), Verlag Chemie Weinheim).

Weiterhin ist die Gerbung unter Verwendung von Aldehyden, insbesondere Dialdehyden wie beispielsweise Glutardialdehyd, bekannt, siehe beispielsweise H. Herfeld, Bibliothek des Leders, Band III, Seite 191, Umschau Verlag Frankfurt/Main, 1984. Nachteilig ist jedoch, dass bei geringen Mengen an Glutardialdehyd, beispielsweise 0,5 bis 0,9 Gew.-% (bezogen auf das Blößengewicht), die Schrumpfungstemperaturen nicht über 70°C hinausgehen und dass sich die erzeugten Halbfabrikate daher nur unzureichend entwässern lassen. Während des Falzens tritt eine Verleimung auf der Fleischseite des Leders auf und beeinflusst die Qualität des Leders nachteilig.

Bei größeren Mengen an eingesetztem Glutardialdehyd können Arbeitssicherheitsprobleme aufgrund der toxischen Eigenschaften des Glutardialdehyds entstehen. Außerdem wird beobachtet, dass man im Allgemeinen ausgegerbte Leder erhält und dass eine anschließende variable Verarbeitung, wie es viele Gerbereien wünschen, nicht mehr möglich ist.

Es ist bekannt, Glutardialdehyd in partiell oder ganz acetalisierter Form zum Gerben einzusetzen, beispielsweise als Methylacetal (Ullmann's Encyclopedia of Industrial Chemistry, Band A15, Seite 259 bis 282 und insbesondere Seite 273 ff., 5. Auflage, (1990), Verlag Chemie Weinheim). Die beschriebenen gegerbten Halbfabrikate neigen jedoch im Allgemeinen rasch zum Vergilben.

In DE-C 38 11 267 wird offenbart, dass eine Acetalisierung von Glutardialdehyd oder anderen Dialdehyden, die 2 bis 8 C-Atome haben, mit kurzkettigen Alkylglykolen, Alkylpolyglykolen, aliphatischen Alkoholen, Glycerin oder Sacchariden vorteilhafte Effekte bringt. Jedoch ist der Dampfdruck der Dialdehyde, die leicht aus den sehr hydrolyseempfindlichen Acetalen rückgebildet wird, noch immer merklich. Auch lassen sich die anwendungstechnischen Eigenschaften der so erhaltenen Leder weiter verbessern.

GB1462309 offenbart Adduhte von Carbonyl-verbindungen mit acetalisiertem 2-Methoxy-2,3-dihydro-4H-pyran als Gerbmittel.

Es bestand daher die Aufgabe, ein neues Verfahren zur Vorgerbung, Gerbung und Nachgerbung von Tierhäuten bereit zu stellen, das die oben genannten Nachteile vermeidet. Insbesondere bestand die Aufgabe, ein Gerbmittel bereit zu stellen, das die oben beschriebenen Nachteile vermeidet.

Demgemäß wurden die eingangs definierten Addukte gefunden. In Formel I 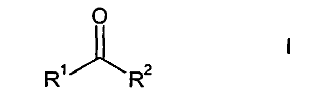 sind die Variablen wie folgt definiert:
R¹, R² sind gleich oder verschieden und gewählt aus Wasserstoff,
C₁-C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl;
C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
   unter substituierten Cycloalkylresten seien beispielhaft genannt: 2-Methylcyclopentyl, 3-Methylcyclopentyl, cis-2,4-Dimethylcyclopentyl, trans-2,4-Dimethyl-cyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,5,5-Tetrachlorcyclohexyl, 2-Thiomethylcyclopentyl, 2-Thiomethylcyclohexyl, 3-Thiomethyl-cyclopentyl, 3-Thiomethylcyclohexyl und weitere Derivate;
C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl,

In einer besonderen Ausführungsform sind die Reste R¹ und R² miteinander unter Bildung eines 4- bis 13-gliedrigen Rings kovalent verbunden. So können R¹ und R² beigemeinsam C₃-C₈-Alkylen, unsubstituiert oder substituiert mit z. B. gemeinsam C₃-C₈-Alkylen, unsubstituiert oder substituiert mit z. B. C₁-C₁₂-Alkyl oder C₆-C₁₄-Aryl, sein. Beispielhaft seien genannt: -(CH₂)₃-, -(CH₂)₂-CH(CH₃)-, -(CH₂)₂-CH(C₂H₅)-, -(CH₂)₂-CH(C₆H₅)-, -(CH₂)₄-, -(CH₂)₅-, -(CH₂)₆-, -(CH₂)₇-, -CH(CH₃)-CH₂-CH₂-CH(CH₃)-, -CH(CH₃)-CH₂-CH₂-CH₂-CH(CH₃)-, vorzugsweise C₃-C₅-Alkylen wie beispielsweise -(CH₂)₃-, -(CH₂)₄-, -(CH₂)₅-.

Ganz besonders bevorzugt sind R¹ und R² jeweils Methyl.

In cyclischen Verbindungen der Formel II 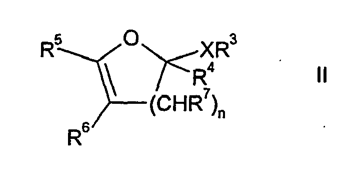 sind die Variablen wie folgt definiert.

X ist ausgewählt aus Sauerstoff, Schwefel oder N-R⁸, wobei Sauerstoff bevorzugt ist.

R³ und R⁸ sind gleich oder verschieden und ausgewählt aus
Wasserstoff,
C₁-C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pen tyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Bu tyl, iso-Butyl, sec.-Butyl und tert.-Butyl, ganz besonders bevorzugt Methyl;
C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl; unter substituierten Cycloalkylgruppen seien beispielhaft genannt: 2-Methylcyclopentyl, 3-Methylcyclopentyl, cis-2,4-Dimethylcyclopentyl, trans-2,4-Dimethyl-cyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlor-cyclohexyl, 2,2,5,5-Tetrachlorcyclohexyl, 2-Thiomethylcyclopentyl, 2-Thio-methylcyclohexyl, 3-Thiomethyl-cyclopentyl, 3-Thiomethylcyclohexyl;
C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl,
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl, unsubstituiert
oder substituiertes C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl, substituiert durch eine oder mehrere
- C₁-C₁₂-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- Halogene wie Fluor, Chlor, Brom und Jod, wobei Chlor und Brom bevorzugt sind,
- C₁-C₁₂-Alkoxygruppen, bevorzugt C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy;
Formyl,
CO-C₁-C₁₂-Alkyl wie Acetyl, Propionyl, n-Butyryl, iso-Butyryl, sec.-Butyryl, tert.-Butyryl, n-Valeroyl, iso-Valeroyl, sec.-Valeroyl, n-Capryl, n-Dodecanoyl; bevorzugt CO-C₁-C₄-Alkyl wie Acetyl, Propionyl, n-Butyryl, iso-Butyryl, sec.-Butyryl, tert.-Butyryl, ganz besonders bevorzugt Acetyl;
CO-C₃-C₁₂-Cycloalkyl wie Cyclopropylcarbonyl, Cyclobutylcarbonyl, Cyclopentylcarbonyl, Cyclohexylcarbonyl, Cycloheptylcarbonyl, Cyclooctylcarbonyl, Cyclononylcarbonyl, Cyclodecylcarbonyl, Cycloundecylcarbonyl und Cyclododecylcarbonyl; bevorzugt sind Cyclopentylcarbonyl, Cyclohexylcarbonyl und Cycloheptylcarbonyl;
unter substituierten Cycloalkylgruppen seien beispielhaft genannt: 2-Methylcyclopentylcarbonyl, 3-Methylcyclopentylcarbonyl, 2-Methylcyclohexylcarbonyl, 3-Methylcyclohexylcarbonyl, 4-Methylcyclohexylcarbonyl, cis-2,5-Dimethylcyclohexylcarbonyl, trans-2,5-Dimethylcyclohexylcarbonyl, 2-Methoxycyclopentylcarbonyl, 2-Methoxycyclohexylcarbonyl, 3-Methoxycyclopentylcarbonyl, 3-Methoxycyclohexylcarbonyl, 2-Chlorcyclopentylcarbonyl, 3-Chlorcyclopentylcarbonyl, 2,4-Dichlorcyclopentylcarbonyl, 2-Chlorcyclohexylcarbonyl, 3-Chlorcyclohexylcarbonyl, 4-Chlorcyclohexylcarbonyl, 2,5-Dichlorcyclohexylcarbonyl, 2-Thiomethylcyclopentylcarbonyl, 2-Thiomethylcyclohexylcarbonyl, 3-Thiomethyl-cyclopentylcarbonyl, 3-Thiomethylcyclohexyl;
CO-C₇-C₁₃-Aralkyl, bevorzugt CO-C₇-C₁₂-Phenylalkyl wie Phenylacetyl, ω-Phenylpropionyl, besonders bevorzugt Phenylacetyl,
C₆-C₁₄-Aryl, beispielsweise Benzoyl, 1-Naphthoyl, 2-Naphthoyl, 1-Anthroyl, 2-Anthroyl, 9-Anthroyl, 1-Phenanthroyl, 2-Phenanthroyl, 3-Phenanthroyl, 4-Phenanthroyl und 9-Phenanthroyl, bevorzugt Benzoyl, 1-Naphthoyl und 2-Naphthoyl, besonders bevorzugt Benzoyl.
R⁴, R⁵, R⁶ gleich oder verschieden und ausgewählt aus Wasserstoff,
C₁-C₁₂Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl, ganz besonders bevorzugt Methyl;
C₃-C₁₂-Cycloalkyl, substituiert oder unsubstituiert, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
unter substituierten Cycloalkylgruppen seien beispielhaft genannt: 2-Methylcyclopentyl, 3-Methylcyclopentyl, cis-2,4-Dimethylcyclopentyl, trans-2,4-Dimethylcyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,5,5-Tetrachlorcyclohexyl, 2-Thiomethylcyclopentyl, 2-Thiomethylcyclohexyl, 3-Thiomethyl-cyclopentyl, 3-Thiomethylcyclohexyl;
C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl,
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl,
unsubstituiert oder substituiertes
C₆-C₁₄-Aryl, beispielsweise Phenyl, 1-naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl, unsubstituiert oder substituiert durch eine oder mehrere
- C₁-C₁₂-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- Halogene wie Fluor, Chlor, Brom und Jod, wobei Chlor und Brom bevorzugt sind,
- C₁-C₁₂-Alkoxygruppen, bevorzugt C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy;
wobei jeweils zwei benachbarte Reste unter Bildung eines Rings miteinander verbunden sein können. So können beispielsweise R⁴ und R⁵ beispielsweise gemeinsam C₁-C₈-Alkylen, unsubstituiert oder substituiert mit z.B. C₁-C₁₂-Alkyl oder C₆-C₁₄-Aryl, sein. Beispielhaft seien genannt: -CH₂-, -CH(CH₃)-, -(CH₂)₂-, -CH₂-CH(CH₃)-, -CH₂-CH(C₂H₅)-, -(CH₂)₃-, -(CH₂)₂-CH(CH₃)-, -(CH₂)₂-CH(C₂H₅)-, -(CH₂)₂-CH(C₆H₅)-, -(CH₂)₄-, -(CH₂)₅-, -(CH₂)₆-, -(CH₂)₇-, -CH(CH₃)-CH₂-CH₂-CH(CH₃)-, -CH(CH₃)-CH₂-CH₂-CH₂-CH(CH₃)-, vorzugsweise C₃-C₅-Alkylen wie beispielsweise -(CH₂)₃-, -(CH₂)₄-, -(CH₂)₅-,
n eine ganze Zahl im Bereich von 1 bis 4, insbesondere 2 oder 3;
R⁷ gleich oder verschieden und ausgewählt aus Wasserstoff,
C₁-C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl, ganz besonders bevorzugt Methyl;
C₃-C₁₂-Cycloalkyl, substituiert oder unsubstituiert, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
unter substituierten Cycloalkylgruppen seien beispielhaft genannt: 2-Methylcyclopentyl, 3-Methylcyclopentyl, cis-2,4-Dimethylcyclopentyl, trans-2,4-Di-methylcyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlor-cyclohexyl, 2,2,5,5-Tetrachlorcyclohexyl, 2-Thiomethylcyclopentyl, 2-Thio-methylcyclohexyl, 3-Thiomethyl-cyclopentyl, 3-Thiomethylcyclohexyl;
C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl,
C₆-C₁₄-Aryl, substituiert oder unsubstituiert, wobei substituierte und unsubstituierte C₆-C₁₄-Arylreste wie oben stehend definiert sind.

In einer Ausführungsform der vorliegenden Erfindung können R⁷ mit R⁶ oder R⁷ mit R⁴ oder R⁷ mit R³ oder für den Fall, dass n größer als 1 ist, jeweils zwei benachbarte Reste R⁷ miteinander unter Bildung eines Rings verbunden sein. So können R⁶ und R⁷ beispielsweise gemeinsam C₁-C₈-Alkylen, unsubstituiert oder substituiert mit C₁-C₁₂-Alkyl oder C₆-C₁₄-Aryl, sein. Beispielhaft seien genannt: -CH₂-, -CH(CH₃)-, -(CH₂)₂-, -CH₂-CH(CH₃)-, -CH₂-CH(C₂H₅)-, -(CH₂)₃-,-(CH₂)₂-CH(CH₃)-, -(CH₂₎₂-CH(C₂H₅)-, -(CH₂)₂-CH(C₆H₅)-, -(CH₂)₄-, -(CH₂)₅-, -(CH₂)₆-, -(CH₂)₇-, -CH(CH₃)-CH₂-CH₂-CH(CH₃)-, -CH(CH₃)-CH₂-CH₂-CH₂-CH(CH₃)-, vorzugsweise C₃-C₅-Alkylen wie beispielsweise -(CH₂)₃-, -(CH₂)₄-, -(CH₂)₅-.

Ganz besonders bevorzugt sind die Reste R⁴ bis R⁷ jeweils Methyl. Ganz besonders bevorzugt wird als Verbindung der allgemeinen Formel II 2-Methoxy-2,3-dihydro-4H-Pyran (Formel II.1) gewählt.

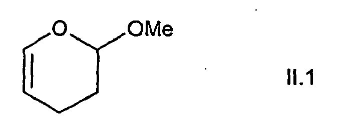

Ganz besonders bevorzugte Carbonylverbindungen der allgemeinen Formel I sind Methylethylketon, Formaldehyd und insbesondere Aceton.

Die erfindungsgemäßen Addukte können in monomerer oder dimerer Form vorliegen. Die erfindungsgemäßen Addukte können aber auch in oligomerer oder polymerer Form vorliegen. Üblicherweise liegen die erfindungsgemäßen Addukte als Gemisch von Dimeren, Oligomeren und Polymeren vor, wobei die Gemische weiterhin herstellungsbedingt Verbindungen der allgemeinen Formel H-X-R³ enthalten können. Auch können die erfindungsgemäßen Addukte im Gemisch mit lagerungsbedingte Verunreinigungen vorliegen, beispielsweise Dehydratisierungsprodukten, Oxidationsprodukten, Hydrolyseprodukten, vernetzten Produkten oder Produkten von einer oder mehreren Retroaldolreaktionen.

Die erfindungsgemäßen Addukte sind herstellbar durch Umsetzung von einer oder mehreren Carbonylverbindungen der allgemeinen Formel I mit einer oder mehreren cyclischen Verbindungen der allgemeinen Formel II.

Ein weiterer Gegenstand der vorliegenden Erfindung ist ein Verfahren zur Herstellung der erfindungsgemäßen Addukte. Das erfindungsgemäße Verfahren geht aus von mindestens einer cyclischen Verbindung der allgemeinen Formel II und mindestens einer Carbonylverbindung der allgemeinen Formel I, die miteinander umgesetzt werden.

In einer Ausführungsform des erfindungsgemäßen Verfahrens setzt man mindestens eine cyclische Verbindung der Formel II mit bis zu 1000 mol-%, bevorzugt mit bis zu 500 mol-% und besonders bevorzugt bis zu 200 mol-% mindestens einer Carbonylverbindungen der allgemeinen Formel I um.

Das erfindungsgemäße Verfahren übt man vorzugsweise bei Temperaturen von 0 bis 120°C, insbesondere 20 bis 85°C aus. Man kann die Umsetzung bei beliebigen Drücken von 0,1 bis 100 bar durchführen, bevorzugt bei Atmosphärendruck. Die Umsetzung erfolgt vorteilhaft in Gegenwart eines Lösemittels, beispielsweise Wasser, Toluol, Petrolether oder n-Heptan, aber der Zusatz von Lösemitteln ist nicht notwendig. Für den Fall, dass die Carbonylverbindung der allgemeinen Formel I unter Reaktionsbedingung flüssig ist, ist die Verwendung von Lösemittel bei der Ausübung des erfindungsgemäßen Verfahrens nicht notwendig.

Man kann das während der Reaktion entstehende Wasser - zusammen mit im Verlaufe der Reaktion gebildetem H-X-R³ - ganz oder zu einem gewissen Anteil abdestillieren.

In einer Ausführungsform der vorliegenden Erfindung führt man das erfindungsgemäße Verfahren bei saurem pH-Wert durch, d.h. beispielsweise bei einem pH-Wert, der im Bereich von 0,5 bis 6,8, bevorzugt 0,7 bis 4 liegt. Vorzugsweise setzt man zur Ausübung des erfindungsgemäßen Verfahrens bei saurem pH-Wert einen oder mehrere saure Katalysatoren ein.

Geeignete saure Katalysatoren sind beispielsweise Phosphorsäure, insbesondere ortho-Phosphorsäure, Ameisensäure, Essigsäure, saure Kieselgele, saures Aluminiumoxid, verdünnte Schwefelsäure, Sulfonsäuren wie beispielsweise Methansulfonsäure oder para-Toluolsulfonsäure. Arbeitet man in nicht-wässrigen Lösemitteln, so ist die Anwendung von P₂O₅ oder Molekularsieb denkbar. Man kann 0,1 bis 20 Gew.-% Katalysator, bezogen auf Carbonylverbindung I, einsetzen.

Als Reaktionszeit für die Bildung der erfindungsgemäßen Addukte sind 10 Minuten bis 24 Stunden sinnvoll, bevorzugt eine bis drei Stunden.

Nach der Reaktion ist es möglich, die durch das erfindungsgemäße Verfahren entstehenden Reaktionsgemische aufzuarbeiten. So kann man eventuell verwendete Lösemittel ganz oder zu einem gewissen Anteil entfernen, beispielsweise durch Destillation, z.B. unter vermindertem Druck. Beispielsweise kann man eventuell verwendete saure Katalysatoren neutralisieren, beispielsweise mit wässriger alkalischer Lösung wie Natronlauge oder Kalilauge. Auch ist es möglich, nicht umgesetzte Ausgangsmaterialien wie z.B. überschüssige Carbonylverbindung der Formel I abzutrennen. Insbesondere in den Fällen, in denen die Carbonylverbindung der allgemeinen Formel I leicht flüchtig ist, beispielsweise Aceton oder Methylethylketon, ist es vorteilhaft, Carbonylverbindungen der allgemeinen Formel I durch Destillation abzutrennen.

In einigen Fällen wird während der Durchführung des erfindungsgemäßen Verfahrens die Bildung eines mehrphasigen Gemischs beobachtet. In den genannten Fällen ist es möglich, die jeweilige wässrige Phase durch beispielsweise Dekantieren oder andere an sich bekannte Methoden zu entfernen.

Bei der Ausübung des erfindungsgemäßen Verfahrens unter den oben beschriebenen Bedingungen kommt es üblicherweise zur Bildung von herstellungsbedingten Neben-und Folgeprodukten, beispielsweise durch Wasserabspaltung (Dehydratisierung), unvollständig abgelaufene Reaktionen, Oxidationen oder auch durch intramolekulare Vernetzung. Bei der Lagerung der erfindungsgemäßen Addukte kann es fernerhin zu lagerungsbedingten Nebenprodukten kommen, beispielsweise durch Wasserabspaltung (Dehydratisierung), Oxidationen oder auch durch Dimerisierung, Oligomerisierung oder Polymerisation sowie durch Vernetzung.

Es ist möglich, die erfindungsgemäße Addukte aufzureinigen und zu isolieren.

Ein weiterer Gegenstand der vorliegenden Erfindung sind Gemische der erfindungsgemäßen Addukte mit oben beschriebenen herstellungsbedingten und/oder lagerungsbedingten Nebenprodukten.

Ein weiterer Gegenstand der vorliegenden Erfindung ist die Verwendung der erfindungsgemäßen Addukte oder der erfindungsgemäßen Gemische zur Herstellung von Halbfabrikaten und von Leder.

In einer Ausführungsform der vorliegenden Erfindung werden die erfindungsgemäßen Addukte oder die erfindungsgemäßen Gemische bei der Vorgerbung, Gerbung oder Nachgerbung von Tierhäuten verwendet.

Weitere Gegenstände der vorliegenden Erfindung sind die Verwendung der erfindungsgemäßen Addukte und der erfindungsgemäßen Gemische zum Vorgerben, Gerben oder Nachgerben von Tierhäuten sowie ein Verfahren zum Vorgerben, Gerben oder Nachgerben von Tierhäuten unter Verwendung der erfindungsgemäßen Addukte oder der erfindungsgemäßen Gemische.

Das erfindungsgemäße Verfahren zum Vorgerben, Gerben oder Nachgerben von Tierhäuten, im Folgenden auch erfindungsgemäßes Gerbverfahren genannt, geht aus von nach konventionellen Methoden vorbehandelten Häuten von Tieren wie beispielsweise Rindern, Schweinen, Ziegen oder Hirschen. Dabei ist es für das erfindungsgemäßen Gerbverfahren nicht wesentlich, ob die Tiere beispielsweise durch Schlachten getötet wurden oder aber an natürlichen Ursachen verendet sind. Zu den konventionellen Methoden der Vorbehandlung gehören das beispielsweise das Äschern, Entkälken, Beizen und Pickeln sowie mechanische Arbeitschritte, beispielsweise zur Entfleischung der Häute.

Das erfindungsgemäße Gerbverfahren wird üblicherweise in Gegenwart von Wasser durchgeführt.

Das erfindungsgemäße Gerbverfahren übt man beispielsweise so aus, dass man ein oder mehrere erfindungsgemäße Addukte gegebenenfalls als Gemisch mit herstellungsbedingten und/oder lagerungsbedingten Nebenprodukten in einer Portion oder in mehreren Portionen unmittelbar vor oder aber während eines Gerbungsschrittes zusetzt. Das erfindungsgemäße Gerbverfahren wird vorzugsweise bei einem pH-Wert von 2,5 bis 4 durchgeführt, wobei man häufig beobachtet, dass der pH-Wert während der Durchführung des erfindungsgemäßen Gerbverfahrens um etwa 0,3 bis drei Einheiten ansteigt. Man kann den pH-Wert auch durch Zugabe abstumpfender Mittel um etwa 0,3 bis drei Einheiten erhöhen.

Das erfindungsgemäße Gerbverfahren übt man im Allgemeinen bei Temperaturen im Bereich von 10 bis 45°C, bevorzugt im Bereich von 20 bis 30°C aus. Bewährt hat sich eine Dauer von 10 Minuten bis 12 Stunden, bevorzugt sind eine bis 3 Stunden. Das erfindungsgemäße Gerbverfahren kann man in beliebigen gerbereiüblichen Gefäßen durchführen, beispielsweise durch Walken in Fässern oder in drehbaren Trommeln mit Einbauten.

In einer Variante des erfindungsgemäßen Gerbverfahrens setzt man die erfindungsgemäßen Addukte oder die erfindungsgemäßen Gemische zusammen mit einem oder mehreren herkömmlichen Gerbstoffen ein, beispielsweise mit Chromgerbstoffen, mineralischen Gerbstoffen, Syntanen, Polymergerbstoffen oder vegetabilen Gerbstoffen, wie sie beispielsweise beschrieben sind in Ullmann's Encyclopedia of Industrial Chemistry, Band A15, Seite 259 bis 282 und insbesondere Seite 268 ff., 5. Auflage, (1990), Verlag Chemie Weinheim. Das Gewichtsverhältnis erfindungsgemäßes Addukt bzw. erfindungsgemäßes Gemisch : herkömmlicher Gerbstoff bzw. Summe der herkömmlichen Gerbstoffe beträgt zweckmäßig von 0,01 : 1 bis 100 : 1, wobei gegebenenfalls vorhandene herstellungsbedingte oder lagerungsbedingte Verunreinigungen der Addukte mitgezählt werden. In einer vorteilhaften Variante des erfindungsgemäßen Verfahrens setzt man nur wenige ppm der herkömmlichen Gerbstoffe den erfindungsgemäßen Addukten. Besonders vorteilhaft ist es jedoch, auf die Beimischung herkömmlicher Gerbstoffe ganz zu verzichten.

In einer Variante des erfindungsgemäßen Gerbverfahrens setzt man ein oder mehrere Addukte gegebenenfalls zusammen mit herstellungsbedingten und/oder lagerungsbedingten Nebenprodukten in einer Portion oder in mehreren Portionen vor oder während des Vorgerbens zu, in einer besonderen Variante bereits im Pickel.

In einer weiteren Variante des erfindungsgemäßen Gerbverfahrens setzt man ein oder mehrere Addukte oder erfindungsgemäße Gemische gegebenenfalls zusammen mit herstellungsbedingten und/oder lagerungsbedingten Nebenprodukten in einer Portion oder in mehreren Portionen vor oder während eines Nachgerbungsschrittes zu. Diese Variante wird im Folgenden auch als erfindungsgemäßes Nachgerbverfahren bezeichnet. Das erfindungsgemäße Nachgerbverfahren geht aus von vorgegerbten Häuten. Diese werden mit den erfindungsgemäßen Addukten behandelt.

Man kann das erfindungsgemäße Nachgerbverfahren unter ansonsten üblichen Bedingungen durchführen. Man wählt zweckmäßig einen oder mehrere, beispielsweise 2 bis 6 Einwirkschritte und kann zwischen den Einwirkschritten mit Wasser spülen. Die Temperatur bei den einzelnen Einwirkschritten beträgt jeweils von 5 bis 60°C, bevorzugt 20 bis 45°C. Man setzt zweckmäßig weitere, während der Nachgerbung üblicherweise verwendete Mittel ein, beispielsweise Fettlicker, Lederfarbstoffe oder Emulgatoren ein.

Ein weiterer Aspekt der vorliegenden Erfindung sind Gerbmittel, enthaltend eine oder mehrere erfindungsgemäße Addukte als aktive Komponente.

Ein weiterer Aspekt der vorliegenden Erfindung sind Halbfabrikate und Leder, hergestellt durch das erfindungsgemäße Verfahren. Die erfindungsgemäßen Leder zeichnen sich durch eine insgesamt vorteilhafte Qualität aus, beispielsweise glatten Narben, homogenere Gerbung über den Querschnitt, verbesserte Reißfestigkeit und Fülle sowie geringere Neigung zum Verfärben, insbesondere zum Vergilben.

In einer speziellen Ausführungsform des erfindungsgemäßen Gerbverfahrens setzt man die erfindungsgemäßen Addukte, gegebenenfalls als Gemische mit herstellungsbedingten oder lagerungsbedingten Nebenprodukten, in Form pulverförmiger Wirkstoffe ein.

Ein weiterer Gegenstand des erfindungsgemäßen Verfahrens betrifft pulverförmige Wirkstoffe, enthaltend 10 bis 100 Gew.-%, bevorzugt 40 bis 90 Gew.-% eines oder mehrere erfindungsgemäße Addukte, gegebenenfalls im Gemisch mit herstellungsbedingten oder lagerungsbedingten Nebenprodukten,
und 0 bis 90 Gew.-%, bevorzugt 10 bis 60 Gew.-% eines oder mehrerer Zuschlagstoffe.

Geeignete Zuschlagstoffe sind in der Regel feste partikuläre Stoffe. Bevorzugt werden sie gewählt aus Stärke, Siliziumdioxid, beispielsweise in der Form von Kieselgel, insbesondere in der Form von sphäroidalen Kieselgelen, Schichtsilikate, Aluminiumoxid sowie Mischoxiden von Silizium und Aluminium.

Die erfindungsgemäßen pulverförmigen Wirkstoffe können aus feinen Partikeln mit einem mittleren Partikeldurchmesser von 100 nm bis 0,1 mm bestehen. Die Partikeldurchmesser folgen dabei einer Partikeldurchmesserverteilung, die eng oder breit sein kann. Auch bimodale Partikelgrößenverteilungen sind denkbar. Die Partikel selbst können irregulär oder sphärischer Form sein, wobei sphärische Partikelformen bevorzugt sind. Die erfindungsgemäßen pulverförmigen Wirkstoffe lassen sich im erfindungsgemäßen Gerbverfahren unter besonders hygienischen Verhältnissen dosieren.

Ein weiterer Gegenstand der vorliegenden Erfindung betrifft ein Verfahren zur Herstellung der erfindungsgemäßen pulverförmigen Wirkstoffe. Das erfindungsgemäße Verfahren geht aus von in Lösung, in Suspension oder Emulsion oder aber von isoliert vorliegenden erfindungsgemäßen Addukten, die herstellungsbedingte oder lagerungsbedingte Nebenprodukte enthalten können. Besonders bevorzugt geht man von Reaktionslösungen aus, wie sie im erfindungsgemäßen Verfahren bei der Herstellung der erfindungsgemäßen Addukte anfallen.

Es hat sich bewährt, die Reaktionslösungen zunächst bis zu einem Restlösemittelgehalt von 50 Gew.-% oder weniger aufzukonzentrieren.

Weiterhin gibt man - so es gewünscht ist - einen oder mehrere Zuschlagstoffe zu.

Anschließend entfernt man die verbliebenen flüchtigen Komponenten. Bevorzugt versprüht man die anfallenden flüssigen, festen oder öligen aufkonzentrierten Reaktionslösungen in einem Sprühtrockner, bevorzugt in einem Sprühturm. Sprühtrockner sind dem Fachmann bekannt und beispielsweise beschrieben in Vauck/Müller, Grundoperationen chemischer Verfahrenstechnik, VCH Weinheim, 1988, 7. Auflage, S. 638-740 und S. 765-766, sowie in der darin zitierten Literatur.

In einer speziellen Ausführungsform des erfindungsgemäßen Gerbverfahrens setzt man die erfindungsgemäßen Addukte, gegebenenfalls als Gemische mit herstellungsbedingten oder lagerungsbedingten Nebenprodukten, in Form von Suspensionen ein, beispielsweise als wässrige Suspensionen.

In einer Ausführungsform des erfindungsgemäßen Gerbverfahrens setzt man ein oder mehrere erfindungsgemäße Addukte, gegebenenfalls als Gemische mit herstellungsbedingten oder lagerungsbedingten Nebenprodukten, in mit polaren Lösemitteln verdünnter Form ein. Geeignet sind beispielsweise Alkohole oder wässrige Alkohole. Als geeignete Alkohole seien Ethylenglykol, Glycerin, Diethylenglykol, Triethylenglykol und Polyethylenglykole sowie Gemische der vorstehenden Alkohole beispielhaft genannt. Als Konzentrationen des oder der polaren Lösungsmittel sind beispielsweise 1 bis 80 Gew.-% geeignet. In einer speziellen Ausführungsform des erfindungsgemäßen Gerbverfahrens setzt man ein oder mehrere erfindungsgemäße Addukte, gegebenenfalls als Gemische mit herstellungsbedingten oder lagerungsbedingten Nebenprodukten, in mit polaren Lösungsmitteln verdünnter Form ein und mischt außerdem Zuschlagstoffe zu. Geeignete Zuschlagstoffe sind beispielsweise Stärke, Siliziumdioxid, Schichtsilikate, Aluminiumoxid sowie Mischoxide von Silizium und Aluminium.

Ein weiterer Gegenstand der vorliegenden Erfindung sind Mischungen der erfindungsgemäßen Addukte, gegebenenfalls als Gemische mit herstellungsbedingten oder lagerungsbedingten Nebenprodukten, mit polaren Lösemitteln wie beispielsweise Alkoholen oder wässrigen Alkoholen.

Ein weiterer Aspekt der vorliegenden Erfindung betrifft die Verwendung der erfindungsgemäßen Addukte, gegebenenfalls als Gemische mit herstellungsbedingten oder lagerungsbedingten Nebenprodukten, zur Konservierung sowie Konservierungsmittel, enthaltend die erfindungsgemäßen Addukte oder die erfindungsgemäßen Gemische. Die erfindungsgemäßen Konservierungsmittel eignen sich zur Konservierung von Erzeugnissen, beispielsweise kosmetischen Erzeugnissen, und von Oberflächen.

Die Erfindung wird durch Beispiele erläutert.

### 1. Herstellung der erfindungsgemäßen Addukte 1.1 bis 1.5

Die Molekulargewichtsbestimmungen erfolgten durch Gelpermeationschromatographie unter den folgenden Bedingungen:
Stationäre Phase: mit Ethylenglykoldimethacrylat vernetztes Hydroxyethylmethacrylatgel, kommerziell erhältlich als: HEMA BIO von FA. PSS, Mainz, Deutschland,
Fluss: 1,5 ml/min, Konzentration: 1 Gew.-% im Laufmittel mit internem Standard, Laufmittel: THF 30 Gew.-%, Acetonitril 10 Gew.-%, 0,1 molare wässrige NaNO₃-Lsg. 60 Gew.-%, interner Standard: 0,01 Gew.-% Benzophenon;
Detektion: UV/vis bei 254 nm.

### 1.1. Herstellung von Addukt 1.1

In einem 1-Liter-Dreihalskolben mit Kühler, Rührer und Thermometer wurden 114 g 2-Methoxy-2,3-dihydro-4H-Pyran (Formel II.1; 1 mol), 114 ml Wasser und 58 g Aceton (1 mol) und 24,6 g einer 85 Gew.-% wässrigen ortho-Phosphorsäure gemischt und 3 Stunden am Rückfluss gekocht. Der pH-Wert betrug 1.

Man erhielt 311 g einer wässrigen Dispersion von Addukt 1.1 in Form eines honigfarbenen Produkts mit breiter Molmassenverteilung (Q = 4,7) und Mₙ 380 g.

### 1.2. Herstellung von Addukt 1.2

In einem 1-Liter-Dreihalskolben mit Kühler, Rührer und Thermometer wurden 128 g 2-Methoxy-2,3-dihydro-4H-Pyran (Formel II.1; 1,12 mol), 128 ml Wasser und 112 g Aceton (2 mol) mit 21 g einer 50 Gew.-% Schwefelsäure vermischt und 3 Stunden am Rückfluss gekocht. Der pH-Wert betrug 0,9.

Anschließend wurde der Kühler gegen eine Destillationsbrücke ausgetauscht und über einen Zeitraum von 3 Stunden bei 70-80 °C und 1 bar wässriges Aceton abdestilliert. Man ließ auf Raumtemperatur abkühlen und stellte mit 25 Gew.-% wässriger Natronlauge einen pH-Wert von 5,2 ein. Danach wurde die Mischung in einen Scheidetrichter überführt und etwa 25 ml einer wässrigen Phase abgetrennt und verworfen. Man erhielt 317 g Addukt 1.2 in Form eines bernsteinfarbenen öligen Produkts mit breiter Molmassenverteilung (Q = 5,1) und Mₙ 610 g.

### 1.3. Herstellung von Addukt 1.3

In einem 1-Liter-Dreihalskolben mit Kühler, Rührer und Thermometer wurden 141 g 2-Methoxy-2,3-dihydro-4H-Pyran (Formel II.1; 1,24 mol), 141 ml Wasser und 83,7 g Aceton (1,44 mol) mit 11 g einer 50 Gew.-% Schwefelsäure vermischt und 3 Stunden am Rückfluss gekocht. Der pH-Wert betrug 0,9.

Anschließend wurde der Kühler gegen eine Destillationsbrücke ausgetauscht und über einen Zeitraum von 3 Stunden bei 70-80 °C und 1 bar wässriges Aceton abdestilliert. Man ließ auf Raumtemperatur abkühlen und stellte mit 25 Gew.-% wässriger Natronlauge einen pH-Wert von 5,2 ein. Anschließend wurden bei 55°C und 450 mbar weitere flüchtige Bestandteile abdestilliert. Man erhielt 297 g Addukt 1.3 in Form eines bernsteinfarbenen öligen Produkts mit breiter Molmassenverteilung (Q = 5,2) mit Mₙ 810 g.

### 1.4. Herstellung von Addukt 1.4

In einem 1-Liter-Dreihalskolben mit Kühler, Rührer und Thermometer wurden 141 g 2-Methoxy-2,3-dihydro-4H-Pyran (Formel II.1; 1,24 mol), 141 ml Wasser und 83,7 g Aceton (1,44 mol) mit 7,5 g einer 70 Gew.-% Methansulfonsäure vermischt und 3 Stunden am Rückfluss gekocht. Der pH-Wert betrug 0,9.

anschließend wurde der Kühler gegen eine Destillationsbrücke ausgetauscht und bei 82°C und Normaldruck flüchtige Bestandteile über einen Zeitraum von 3 Stunden wässriges Aceton abdestilliert.

Man ließ auf Raumtemperatur abkühlen und stellte mit 6 ml 50 Gew.-% wässriger Natronlauge einen pH-Wert von 5,3 ein. Anschließend wurden bei 55°C und 550 mbar über einen Zeitraum von einer Stunde weitere flüchtige Bestandteile abdestilliert. Man erhielt 280 g Addukt 1.4 in Form einer gelben öligen Flüssigkeit mit breiter Molmassenverteilung (Q = 3,8) mit Mₙ 840 g.

### 1.5. Herstellung von Addukt 1.5

In einem 1-Liter-Dreihalskolben mit Kühler, Rührer und Thermometer wurden 141 g 2-Methoxy-2,3-dihydro-4H-Pyran (Formel II.1; 1,24 mol), 144,3 g wässrige Formalinlösung (1,44 mol Formaldehyd) und 45 ml Wasser mit 7,5 g einer 70 Gew.-% Methansulfonsäure vermischt und 3 Stunden am Rückfluss gekocht. Der pH-Wert betrug 0,9. Anschließend wurde der Kühler gegen eine Destillationsbrücke ausgetauscht und bei 75°C und Normaldruck über einen Zeitraum von 3 Stunden wässriges Aceton abdestilliert.

Man ließ auf Raumtemperatur abkühlen und stellte mit etwa 9 ml 50 Gew.-% wässriger Natronlauge einen pH-Wert von 4,2 ein. Anschließend wurden bei 49°C und 35 mbar weitere flüchtige Bestandteile abdestilliert. Man erhielt 151 g Addukt 1.5 in Form eines öligen braunen Produkts mit breiter Molmassenverteilung (Q = 4,6) mit Mₙ 1120 g.

### 2. Gerbversuche

### 2.1. Erfindungsgemäßer Gerbversuch 2.1

Angaben in Gew.-% beziehen sich auf das Pickelgewicht, wenn nicht anders angegeben.

Streifen mit einem Gewicht von je 2500 g gepickelter Rindsblöße mit einer Spaltstärke von 2,5 mm wurden bei einem pH-Wert von 3,0-3,2 und 25°C in einem 10-I-Fass mit 750 ml Wasser und, bezogen auf die Pickelblöße, 3 Gew.-% der Addukt 1.1 versetzt. Nach einer Walkzeit von 60 Minuten wurden 2 Gew.-% des Sulfongerbstoffs Basyntan® SW (BASF Aktiengesellschaft) zugesetzt und weitere 2 Stunden gewalkt. Anschließend wurde mit 0,5 Gew.-% Magnesiumoxid innerhalb von 6 Stunden der pH-Wert auf 4,9-5,1 gestellt. Die Flotte wurde abgelassen und die Haut mit 300 ml Wasser gewaschen. Nach dem Abwelken wurden die Häute auf 1,6-1,8 mm gefalzt. Man erhielt das erfindungsgemäße Halbfabrikat 2.1.

Die Falzbarkeit wurde durch Versuche an einer Falzmaschine bestimmt. Falzmaschinen arbeitet mit rotierenden Messern. Bei schlechter Falzbarkeit schmierten die Messer über die Oberfläche, es kam zu einer Temperaturerhöhung auf der Oberfläche der Leder, so dass ein homartiges Aufschmelzen die Haut irreversibel schädigte. Die Benotung erfolgte nach einem Notensystem von 1 (sehr gut) bis 5 (mangelhaft).

2.2 bis 2.5. Erfindungsgemäße Gerbversuche 2.2 bis 2.5 und Vergleichsversuch V 2.6

Der oben beschriebene Gerbversuch wurde wiederholt, jedoch wurde das erfindungsgemäße Addukt 1.1 durch die erfindungsgemäßen Addukte 1.2, 1.3, 1.4 bzw. 1.5 ersetzt. Man erhielt dementsprechend die erfindungsgemäßen Halbfabrikate 2.2, 2.3, 2.4 bzw. 2.5.

Der Vergleichsversuch V 2.6 wurde analog durchgeführt, jedoch wurde das erfindungsgemäße Addukt durch 3 Gew.-% Glutaraldehyd (50 Gew.-% wässrige Lösung) ersetzt. Man erhielt das Vergleichs-Halbfabrikat V 2.6.

Die Schrumpftemperaturen wurden gemäß der Vorschrift aus DIN 53 336 (Jahr 1977) bestimmt, wobei die Vorschrift wie folgt modifiziert wurde:
Punkt 4.1: Die Probestücke hatten die Abmessungen 3 cm · 1 cm; die Dicke wurde nicht bestimmt.
Punkt 4.2: es wurde nur eine anstatt 2 Proben pro Ledermuster geprüft.
Punkt 6: entfiel
Punkt 7: die Trocknung im Vakuumexsikkator entfiel.
Punkt 8: bei Rückgang des Zeigers wurde die Schrumpfungstemperatur gemessen.

Die Benotung der Falzbarkeit und der Vergilbung erfolgte wie in der Schule: 1 sehr gut, 2 gut, 3 befriedigend, 4 ausreichend.

**Tabelle 1: Ergebnis der Gerbung und analytische Bewertung der erfindungsgemäßen Halbfabrikate**

| Nummer | Halbfabrikat | Falzbarkeit | Schrumpftemperatur | Vergilbung |
|---|---|---|---|---|
| | | | [°C] | |
| 2.1 | 2.1 | 3 | 74,5 | 2 |
| 2.2 | 2.2 | 2,5 | 76 | 2,5 |
| 2.3 | 2.3 | 2 | 77,5 | 2,5 |
| 2.4 | 2.4 | 2 | 79 | 2 |
| 2.5 | 2.5 | 3 | 74 | 3,5 |
| V 2.6 | Glutaraldehyd | 3 | 77 | 4 |

### 3. Herstellung erfindungsgemäßer Leder und Vergleichsversuch

Angaben in Gew.-% beziehen sich auf das Falzgewicht, wenn nicht anders angegeben.

### 3.1. Herstellung des Leders 3.1 aus Halbfabrikat 2.1

1800 g Halbfabrikat 2.1 wurde zusammen mit den folgenden Agenzien 20 Minuten gewalkt:
120 Gew.-% Wasser, 5 Gew.-% des Sulfongerbstoffs Basyntan® SW (BASF Aktiengesellschaft) und
4 % einer 30 Gew.-% wässrigen, mit NaOH teilneutralisierten Lösung eines Methacrylsäure-Homopolymers mit den folgenden analytischen Daten: Mₙ ca. 10.000; K-Wert nach Fikentscher: 12 (bestimmt als 1 Gew.-% wässrige Lösung), Viskosität der 30 Gew.-% Lösung: 65 mPa·s (DIN EN ISO 3219, 23°C), pH-Wert 5,1.
Dann wurden 6 Gew.-% des Vegetabilgerbstoffs Tara® (BASF Aktiengesellschaft) und 2 Gew.-% des Harzgerbstoffs Relugan® S (BASF Aktiengesellschaft) sowie 2 Gew.-% Farbstoff Luganil® braun NGB dosiert und die Mischung gewalkt. Nach zwei Stunden wurde mit Ameisensäure der pH-Wert 3,6 eingestellt. Als Fettungskomponente wurden 6 Gew.-% Lipodermliquer CMG® (BASF Aktiengesellschaft) und 1 Gew.-% Lipamin OK® (BASF Aktiengesellschaft) zugesetzt. Nach einer Walkzeit von weiteren 60 Minuten wurde mit Ameisensäure der pH-Wert 3,2 eingestellt. Vor dem Ablassen der Flotte wurde eine Probe der Flotte gezogen. Man ließ die Flotte ab.

Das so erhaltene Leder wurde zweimal mit je 100 Gew.-% Wasser gewaschen, über Nacht feucht gelagert und nach dem Abwalken auf dem Spannrahmen bei 50 °C getrocknet. Man erhielt Leder 3.1. Nach dem Stollen wurde Leder 3.1 wie unten stehend beurteilt.

Die Bewertung erfolgte nach einem Notensystem von 1 (sehr gut) bis 5 (mangelhaft). Die Bewertung der Flottenauszehrung erfolgte visuell nach den Kriterien Restfarbstoff (Extinktion) und Trübung (Fettungsmittel), aus denen der Mittelwert gebildet wurde.

### Beispiele 3.2 bis 3.5, Vergleichsbeispiel V 3.6

Das obige Beispiel wurde wiederholt, jedoch jeweils mit den erfindungsgemäßen Halbfabrikaten 2.2 bis 2.5. Für das Vergleichsbeispiel V3.6 wurde das Halbfabrikat aus Vergleichsbeispiel V2.6 weiter verarbeitet. Die Bewertung der anwendungstechnischen Eigenschaften findet sich in Tabelle 2.

**Tabelle 2**

| Leder | Flottenauszehrung | Fülle | Narben-festigkeit | Weich | Zugfestigkeit | Stichausreißkraft | Egalität |
|---|---|---|---|---|---|---|---|
| | | | | heit | [N] | [N] | |
| 3.1 | 3 | 2,5 | 3 | 3,5 | 256 | 196 | 2,5 |
| 3.2 | 2,5 | 2,5 | 2 | 3 | 270 | 194 | 2,5 |
| 3.3 | 2 | 1,5 | 2,5 | 2 | 283 | 201 | 1 |
| 3.4 | 1,5 | 1 | 1,5 | 2 | 287 | 229 | 1 |
| 3.5 | 1,5 | 2,5 | 2 | 2,5 | 260 | 218 | 1,5 |
| V 3.6 | 3 | 3,5 | 3,5 | 3 | 247 | 191 | 3 |

Die Zugfestigkeit wurde nach DIN 53328 bestimmt.

Die Stichausreißkraft wurde nach DIN 53331 bestimmt.

## Patentansprüche

1. Addukte, erhältlich durch Umsetzung von Carbonylverbindungen der allgemeinen Formel I, 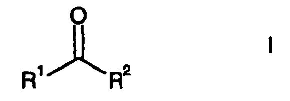 in denen die Variablen wie folgt definiert sind:
R¹, R² ausgewählt aus Wasserstoff, C₁-C₁₂-Alkyl, C₃-C₁₂-Cycloalkyl, substituiert oder unsubstituiert, C₇-C₁₃-Aralkyl, wobei R¹ und R² miteinander unter Bildung eines Rings verbunden sein können,
mit cyclischen Verbindungen der allgemeinen Formel II, 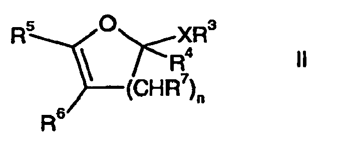
in denen die Variablen wie folgt definiert sind:
X ausgewählt aus Sauerstoff, Schwefel und N-R⁸,
R³, R⁸ gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, Formyl, CO-C₁-C₁₂-Alkyl, CO-C₃-C₁₂-Cycloalkyl, substituiert oder unsubstituiert, CO-C₇-C₁₃-Aralkyl, CO-C₆-C₁₄-Aryl, wobei für den Fall, dass X für N-R⁸ steht, R³ und R⁸ unter Bildung eines Rings miteinander verbunden sein können;
R⁴, R⁵, R⁶ gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, wobei jeweils zwei benachbarte Reste unter Bildung eines Rings miteinander verbunden sein können;
n eine ganze Zahl im Bereich von 1 bis 4;
R⁷ gleich oder verschieden und ausgewählt aus Wasserstoff, C₁-C₁₂-Alkyl, C₃-C₁₂-Cycloalkyl, substituiert oder unsubstituiert, C₇-C₁₃-Aralkyl, C₆-C₁₄-Aryl, substituiert oder unsubstituiert, wobei R⁷ mit R⁵ oder auch jeweils zwei benachbarte Reste R⁷ miteinander unter Bildung eines Rings verbunden sein können.

2. Addukte nach Anspruch 1, **dadurch gekennzeichnet, dass** X Sauerstoff bedeutet.

3. Addukte nach Anspruch 1 oder 2, **dadurch gekennzeichnet, dass** R⁴ bis R⁷ jeweils Wasserstoff bedeuten.

4. Gemische, enthaltend mindestens ein Addukt nach Anspruch 1 bis 3 mit herstellungs- oder lagerungsbedingten Nebenprodukten.

5. Verfahren zur Herstellung von Addukten nach Anspruch 1 bis 3 oder Gemischen nach Anspruch 4, **dadurch gekennzeichnet, dass** man Carbonylverbindungen der allgemeinen Formel I mit cyclischen Verbindungen der allgemeinen Formel II umsetzt.

6. Verfahren nach Anspruch 5, **dadurch gekennzeichnet, dass** man die Umsetzung bei saurem pH-Wert durchführt.

7. Verwendung von Addukten nach den Ansprüchen 1 bis 3 oder Gemischen nach Anspruch 4 zur Herstellung von Halbfabrikaten und von Leder.

8. Verfahren zur Herstellung von Halbfabrikaten und Leder, **dadurch gekennzeichnet, dass** man Addukte nach den Ansprüchen 1 bis 3 oder Gemische nach Anspruch 4 einsetzt.

9. Verfahren nach Anspruch 8, **dadurch gekennzeichnet, dass** man Addukte nach den Ansprüchen 1 bis 3 oder Gemische nach Anspruch 4 in mit polaren Lösemitteln verdünnter Form einsetzt.

10. Verfahren nach Anspruch 9, **dadurch gekennzeichnet, dass** man ein oder mehrere Addukte nach den Ansprüchen 1 bis 3 oder Gemische nach Anspruch 4, als pulverförmigen Wirkstoff einsetzt.

11. Pulverförmiger Wirkstoff, enthaltend
10 bis 100 Gew.-% eines oder mehrerer Addukte nach Anspruch 1 bis 3 oder Gemische nach Anspruch 4,
und 0 bis 90 Gew.-% eines oder mehrerer Zuschlagstoffe.

12. Pulverförmiger Wirkstoff nach Anspruch 11, in dem der oder die Zuschlagstoffe gewählt werden aus Stärke, Siliziumdioxid, Schichtsilikaten, Aluminiumoxid sowie Mischoxiden von Silizium und Aluminium.

13. Verfahren zur Herstellung von pulverförmigen Wirkstoffen nach Anspruch 11 oder 12, **dadurch gekennzeichnet, dass** man sie durch Sprühtrocknung gewinnt.

14. Verfahren nach Anspruch 8, **dadurch gekennzeichnet, dass** man ein oder mehrere Addukte nach den Ansprüchen 1 bis 3 oder Gemische nach Anspruch 4 in mit polarem Lösemittel verdünnter Form in Anwesenheit von Zuschlagstoffen einsetzt.

15. Halbfabrikate und Leder, hergestellt nach einem Verfahren nach Anspruch 8 bis 9 oder 14.

16. Verwendung von Addukten nach Anspruch 1 bis 3 oder von Gemischen nach Anspruch 4 als Konservierungsmittel.

17. Verwendung von pulverförmigen Wirkstoffen nach Anspruch 11 oder 12 als Konservierungsmittel.

## Claims

1. An adduct obtainable by reacting carbonyl compounds of the formula I 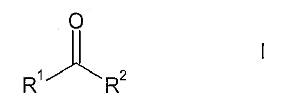 where
R¹ and R² are selected from hydrogen, C₁-C₁₂-alkyl, C₃-C₁₂-cycloalkyl, substituted or unsubstituted, C₇-C₁₃-aralkyl, it being possible for R¹ and R² to be linked to one another with formation of a ring,
with cyclic compounds of the formula II 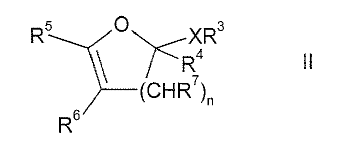 where
X is selected from oxygen, sulfur and N-R⁸,
R³ and R⁸ are identical or different and are selected from hydrogen, C₁-C₁₂-alkyl, C₃-C₁₂-cycloalkyl, substituted or unsubstituted, C₇-C₁₃-aralkyl, C₆-C₁₄-aryl, substituted or unsubstituted, formyl, CO-C₁-C₁₂-alkyl, CO-C₃-C₁₂-cycloalkyl, substituted or unsubstituted, CO-C₇-C₁₃-aralkyl, CO-C₆-C₁₄-aryl, where when X is N-R⁸, R³ and R⁸ may be linked to one another with formation of a ring;
R⁴, R⁵ and R⁶ are identical or different and are selected from hydrogen, C₁-C₁₂-alkyl, C₃-C₁₂-cycloalkyl, substituted or unsubstituted, C₇-C₁₃-aralkyl, C₆-C₁₄-aryl, substituted or unsubstituted, it being possible in each case for two neighboring radicals to be linked to one another with formation of a ring;
n is an integer in the range from 1 to 4;
R⁷ are identical or different and are selected from hydrogen, C₁-C₁₂-alkyl, C₃-C₁₂-cycloalkyl, substituted or unsubstituted, C₇-C₁₃-aralkyl, C₆-C₁₄-aryl, substituted or unsubstituted, it being possible for R⁷ to be linked to R⁶ or in each case two neighboring radicals R⁷ to be linked to one another with formation of a ring.

2. The adduct according to claim 1, wherein X is oxygen.

3. The adduct according to claim 1 or 2, wherein R⁴ to R⁷ are each hydrogen.

4. A mixture comprising at least one adduct according to any of claims 1 to 3 with byproducts arising from the preparation or storage.

5. A process for the preparation of an adduct according to any of claims 1 to 3 or a mixture according to claim 4, wherein carbonyl compounds of the formula I are reacted with cyclic compounds of the formula II.

6. The process according to claim 5, wherein the reaction is carried out at acidic pH.

7. The use of an adduct according to any of claims 1 to 3 or a mixture according to claim 4 for the production of semifinished products and of leather.

8. A process for the production of semifinished products and leather, wherein an adduct according to any of claims 1 to 3 or a mixture according to claim 4 is used.

9. The process according to claim 8, wherein an adduct according to any of claims 1 to 3 or a mixture according to claim 4 is used in a form diluted with a polar solvent.

10. The process according to claim 9, wherein one or more adducts according to any of claims 1 to 3 or mixtures according to claim 4 are used as an active ingredient in powder form.

11. An active ingredient in powder form, comprising
from 10 to 100% by weight of one or more adducts according to any of claims 1 to 3 or a mixture according to claim 4
and from 0 to 90% by weight of one or more additives.

12. The active ingredient in powder form according to claim 11, in which the additive or additives is or are selected from starch, silica, sheet silicates, alumina and mixed oxides of silicon and aluminum.

13. A process for the preparation of an active ingredient in powder form according to claim 11 or 12, wherein said active ingredient is obtained by spray-drying.

14. The process according to claim 8, wherein one or more adducts according to any of claims 1 to 3 or mixtures according to claim 4 are used in a form diluted with a polar solvent, in the presence of additives.

15. A semifinished product or a leather, produced by a process according to claim 8 or 9 or 14.

16. The use of an adduct according to any of claims 1 to 3 or of a mixture according to claim 4 as a preservative.

17. The use of an active ingredient in powder form according to claim 11 or 12 as a preservative.

## Revendications

1. Produits d'addition, pouvant être obtenus par transformation de composés carbonyle de formule générale I, 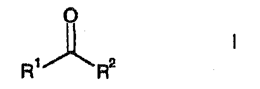 où les variables sont définies comme suit :
R¹, R² sont choisis parmi hydrogène, C₁-Cₗ₂-alkyle, C₃-C₁₂-cycloalkyle substitué ou non substitué, C₇-C₁₃-aralkyle, où R¹ et R² peuvent être liés l'un à l'autre en formant un cycle,
avec des composés cycliques de formule générale II, 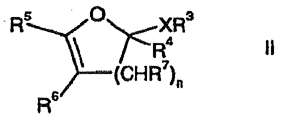
où les variables sont définies comme suit :
X est choisi parmi l'oxygène, le soufre et N-R⁸,
R³, R⁸ sont identiques ou différents et choisis parmi hydrogène, C₁-C₁₂-alkyle, C₃-C₁₂-cycloalkyle substitué ou non substitué, C₇-C₁₃-aralkyle; C₆-C₁₄-aryle substitué ou non substitué, formyle, CO-C₁-C₁₂-alkyle, CO-C₃-C₁₂-cycloalkyle substitué ou non substitué, CO-C₇-C₁₃-aralkyle, CO-C₆-C₁₄-aryle, où, pour le cas où X représente N-R⁸, R³ et R⁸ peuvent être liés l'un à l'autre en formant un cycle ;
R⁴, R⁵, R⁶ sont identiques ou différents et choisis parmi hydrogène, C₁-C₁₂-alkyle, C₃-C₁₂-cycloalkyle substitué ou non substitué, C₇-C₁₃-aralkyle, C₆-C₁₄-aryle substitué ou non substitué, où à chaque fois deux radicaux adjacents peuvent être liés l'un à l'autre en formant un cycle ;
n vaut un nombre entier de la plage de 1 à 4 ;
R⁷ est identique ou différent et choisi parmi hydrogène, C₁-C₁₂-alkyle, C₃-C₁₂-cycloalkyle substitué ou non substitué, C₇-C₁₃-aralkyle, C₆-C₁₄-aryle substitué ou non substitué, où R⁷ peut être lié à R⁶ ou également à chaque fois deux radicaux R⁷ adjacents peuvent être liés l'un à l'autre en formant un cycle.

2. Produits d'addition selon la revendication 1, **caractérisés en ce que** X signifie oxygène.

3. Produits d'addition selon la revendication 1 ou 2, **caractérisés en ce que** R⁴ à R⁷ signifient à chaque fois hydrogène.

4. Mélanges, contenant au moins un produit d'addition selon la revendication 1 à 3 avec des produits secondaires provoqués par la préparation ou l'entreposage.

5. Procédé pour la préparation de produits d'addition selon la revendication 1 à 3 ou de mélanges selon la revendication 4, **caractérisé en ce qu'**on transforme des composés carbonyle de formule générale I avec des composés cycliques de formule générale II.

6. Procédé selon la revendication 5, **caractérisé en ce qu'**on réalise la transformation à un pH acide.

7. Utilisation de produits d'addition selon les revendications 1 à 3 ou de mélanges selon la revendication 4 pour la fabrication de produits semi-finis et de cuir.

8. Procédé pour la fabrication de produits semi-finis et de cuir, **caractérisé en ce qu'**on utilise des produits d'addition selon les revendications 1 à 3 ou des mélanges selon la revendication 4.

9. Procédé selon la revendication 8, **caractérisé en ce qu'**on utilise des produits d'addition selon les revendications 1 à 3 ou des mélanges selon la revendication 4 sous une forme diluée avec des solvants polaires.

10. Procédé selon la revendication 9, **caractérisé en ce qu'**on utilise un ou plusieurs produits d'addition selon les revendications 1 à 3 ou des mélanges selon la revendication 4 sous forme de substance active pulvérulente.

11. Substance active pulvérulente, contenant
- 10 à 100% en poids d'un ou de plusieurs produits d'addition selon la revendication 1 à 3 ou des mélanges selon la revendication 4,
- et 0 à 90% en poids d'un ou de plusieurs adjuvants.

12. Substance active pulvérulente selon la revendication 11, où le ou les adjuvants sont choisis parmi l'amidon, le dioxyde de silicium, les silicates à couches, l'oxyde d'aluminium ainsi que les oxydes mixtes de silicium et d'aluminium.

13. Procédé pour la préparation de substances actives pulvérulentes selon la revendication 11 ou 12, **caractérisé en ce qu'**on les obtient par séchage par pulvérisation.

14. Procédé selon la revendication 8, **caractérisé en ce qu'**on utilise un ou plusieurs produits d'addition selon les revendications 1 à 3 ou des mélanges selon la revendication 4 sous une forme diluée avec des solvants polaires en présence d'adjuvants.

15. Produits semi-finis et cuir, préparés selon un procédé selon la revendication 8 à 9 ou 14.

16. Utilisation de produits d'addition selon la revendication 1 à 3 ou de mélanges selon la revendication 4 comme conservateurs.

17. Utilisation de substances actives pulvérulentes selon la revendication 11 ou 12 comme conservateur.
